(19) Europäisches Patentamt
European Patent Office
Office européen des brevets

(11) Veröffentlichungsnummer: **0 280 268**
**A1**

(12) **EUROPÄISCHE PATENTANMELDUNG**

(21) Anmeldenummer: **88102715.5**

(22) Anmeldetag: **24.02.88**

(51) Int. Cl.⁴: **C07D 495/04**, A61K 31/38, //(C07D495/04,335:00,333:00)

Patentansprüche für folgende Vertragsstaaten: ES + GR.

(30) Priorität: **27.02.87 US 20053**

(43) Veröffentlichungstag der Anmeldung: **31.08.88 Patentblatt 88/35**

(84) Benannte Vertragsstaaten: **AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Hutchison, Alan J.**
**35 Lynwood Road**
**Verona New Jersey 07044(US)**

(74) Vertreter: **Zumstein, Fritz, Dr. et al**
**Bräuhausstrasse 4**
**D-8000 München 2(DE)**

(54) **Thienothiopyrane.**

(57) Verbindungen der Formel I

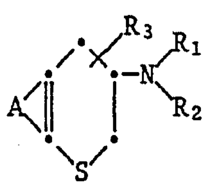

(I),

worin A einen divalenten Radikal $-S-CR_4=CR_5-$, worin $R_4$ und $R_5$ unabhängig voneinander Wasserstoff oder Niederalkyl, $R_1$ Wasserstoff, Niederalkyl oder Arylniederalkyl, $R_2$ Wasserstoff, Niederalkyl oder Arylniederalkyl, oder $R_1$ und $R_2$ zusammen Alkylen mit 4 bis 6 C-Atomen, $R_3$ Wasserstoff oder Niederalkyl bedeuten; S-Oxide davon; und pharmazeutisch annehmbare Salze davon, welche als präsynaptische Dopamin-Rezeptor-Agonisten für die Behandlung von Störungen des zentralen Nervensystems verwendet werden können. Sie werden beispielsweise hergestellt, indem man eine Verbindung der Formel II

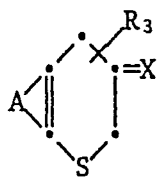

(II),

mit einer Verbindung der Formel III

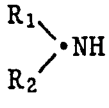

(III),

EP 0 280 268 A1

kondensiert.

# Thienothiopyrane

Die vorliegende Erfindung betrifft neue 5,6-Dihydro-4H-thieno [2,3-b]thiopyran-5-amine und 6,7-Dihydro-5H-thieno[3,2-b]thiopyran-6-amine, welche als Modulatoren für die Rezeptoren des zentralen Nervensystems verwendbar sind, insbesondere als präsynaptische Dopaminrezeptor-Agonisten für die Behandlung von Störungen des zentralen Nervensystems, Verfahren zur Herstellung dieser Verbindungen, pharmazeutische Zusammensetzungen, welche diese Verbindungen enthalten, Methoden zur Stimulierung von präsynaptischen Dopaminrezeptoren in Säugern und zur Behandlung von Syndromen, Zuständen und Krankheiten in Säugern durch Anwendung der genannten Verbindungen oder einer pharmazeutischen Zusammensetzung enthaltend die genannten Verbindungen.

Die Erfindung betrifft neue Dihydro-thienothiopyran-Derivate der Formel I

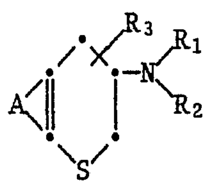

(I),

worin A einen divalenten Radikal $-S-CR_4=CR_5-$, worin $R_4$ und $R_5$ unabhängig voneinander Wasserstoff oder Niederalkyl, $R_1$ Wasserstoff, Niederalkyl oder Arylniederalkyl, $R_2$ Waserstoff, Niederalkyl oder Arylniederalkyl, oder $R_1$ und $R_2$ zusammen Alkylen mit 4 bis 6 C-Atomen, und $R_3$ Wasserstoff oder Niederalkyl bedeuten; S-Oxide davon; und pharmazeutisch annehmbare Salze dieser Verbindungen.

Die Erfindung betrifft insbesondere neue 5,6-Dihydro-4H-thieno[2,3-b]thiopyran-Derivate der Formel Ia

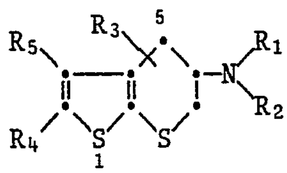

(Ia),

worin $R_1$, $R_2$, $R_3$, $R_4$ und $R_5$ die oben genannten Bedeutungen haben; S-Oxide davon, und pharmazeutisch annehmbare Salze dieser Verbindungen.

Ein weiterer bevorzugter Erfindungsgegenstand betrifft neue 6,7-Di-hyro-5H-thieno[3,2-b]thiopyran-Derivate der Formel Ib

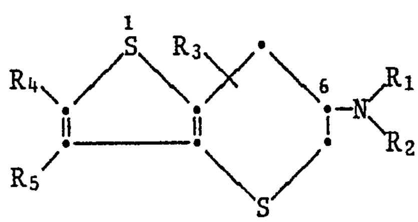

(Ib),

worin $R_1$, $R_2$, $R_3$, $R_4$ und $R_5$ die oben genannten Bedeutungen haben; S-Oxide davon, und pharmazeutisch annehmbare Salze dieser Verbindungen.

Bevorzugt sind Verbindungen der Formel I, Ia oder Ib, worin $R_1$ und $R_2$ unabhängig voneinander Wasserstoff, Niederalky oder Arylniederalkyl bedeuten, oder $R_1$ und $R_2$ zusammengenommen Butylen oder Pentylen darstellen; $R_3$, $R_4$ und $R_5$ Wasserstoff oder Niederalkyl bedeuten; und pharmazeutisch annehmbare Salze dieser Verbindungen.

Weiterhin sind Verbindungen der Formel I, Ia oder Ib bevorzugt, worin $R_1$ Niedralkyl, $R_2$ Niederalkyl oder Arylniederalkyl; $R_3$, $R_4$ und $R_4$ Wasserstoff bedeuten; und pharmazeutisch annehmbare Salze dieser Verbindungen.

Bevorzugt sind Verbindungen der Formel I, Ia oder Ib, worin $R_1$ und $R_2$ Niederalkyl bedeuten, $R_3$, $R_4$ und $R_5$ Wasserstoff bedeuten; und pharmazeutisch annehmbare Salze dieser Verbindungen.

Bevorzugt sind auch Verbindungen der Formel I, Ia oder Ib, worin $R_1$ und $R_2$ vorzugsweise identisch

sind und einen geradkettigen $C_1$-$C_4$-Alkylrest bedeuten; $R_3$, $R_4$ und $R_5$ Wasserstoff bedeuten; und pharmazeutisch annehmbare Salze dieser Verbindungen.

Besonders bevorzugt sind Verbindungen der Formel Ia.

Die allgemeinen Definitionen in der Beschreibung dieser Erfindung haben die folgenden Bedeutungen:

Der weiter vorn und im folgenden verwendete Ausdruck "Nieder" bedeutet, dass so bezeichnete organische Reste oder Verbindungen bis einschliesslich 7 und vorzugsweise einschliesslich 4 und insbesondere 1 oder 2 C-Atome, enthalten.

Niederalkyl enthält 1 bis 7 C-Atome, vorzugsweise 1 bis 4 C-Atome, insbesondere geradkettige Reste und bedeutet beispielsweise Methyl, Ethyl, Propyl oder Butyl.

Alkylen enthält 4 bis 6 C-Atome (für $R_1$ und $R_2$ zusammen) insbesondere geradkettige Reste, und bedeutet beispielsweise Butylen, Pentylen und Hexylen und gebunden mit Stickstoffatoms bildet Pyrrolidino, Piperidino und Perhydroazepino.

Aryl ist ein carbocyclischer oder aromatischer heterocyclisher Rest.

Aryl ist vorzugsweise Phenyl oder Phenyl substituiert durch 1-3 Niederalkyl, Halogen oder Niederalkoxy; Aryl bedeutet ebenfalls Heteroaryl, z.B. Pyridyl oder Thienyl.

Arylniederalkyl ist beispielsweise Benzyl oder 2-Phenylethyl, welche am Phenyl durch die unter der Definition für Aryl genannten Substituenten substituiert sein können.

S-Oxide sind mono-S-Oxide (Sulfoxid) oder di-S-Oxide (Sulfonyl) Derivate, insbesondere an dem Schwefelatom in dem Thiopyranring.

Abhängig von der Natur der Substituenten und der dadurch sich ergebenden Anzahl an asymmetrischen C-Atomen können die erfindungsgemässen Verbindungen in Form von Stereoisomeren, z.B. optisch reinen Antipoden, Diastereomeren oder Racematen, vorliegen, welche sämtlich Gegenstand der vorliegenden Erfindung sind.

Pharmazeutisch annehmbare Salse sind therapeutisch verwendbare Säureadditionssalze, vorzugsweise Salze von anorganischen oder organischen Säuren, z.B. starken Mineralsäuren, z.B. Halogenwasserstoffsäuren, z.B. Chlorwasserstoff-oder Bromwasserstoffsäure, Schwefel-, Phosphor-oder Salpetersäure, aliphatischen oder aromatischen Carbonsäuren oder Sulfonsäuren, z.B. Ameisen-, Essig-, Propion-, Bernstein-, Glycol-, Milch-, Apfel-, Wein-, Glucon-, Zitronen-, Malein-, Fumar-, Brenztrauben-, Phenylessig-Benzoe-, 4-Aminosalicyl-, Pamoa-, Nicotin-, Methansulfon-, Ethansulfon-, Hydroxyethansulfon-, Benzolsulfon-, p-Toluolsulfon-, Naphthalinsulfon-, Sulfanil-, Cyclohexylsulfamin-oder Ascorbinsäure.

Die neuen erfindungsgemässen Verbindungen der Formel I sind in bekannten pharmakologischen in-situ und in-vivo Modellen als präsynaptische Dopamin-Rezeptor Agonisten wirksam. Selektive Dopamin-Rezeptor Agonisten können bei der Behandlung von z.B. Dyskinese, Parkinsonismus oder psychischen Störungen, wie Schizophrenie, verwendet werden.

Die genannten Eigenschaften lassen sich in in-vivo und in-vitro Versuchen, vorzugsweise bei Säugetieren, z.B. Ratten, Hunden oder Affen, aber auch an entnommenen isolierten Organen und Gewebeproben nachweisen. Die Verbindung können in-vitro in Form von Lösungen, z.B. wässrigen Lössungen, und in-vivo entweder enteral oder parenteral, mit Vorteil oral oder intravenös, z.B. in Gelatinekapseln, als Stärkesuspensionen oder in wässrigen Lösungen appliziert werden. Der Dosisbereich in-vitro kann zwischen $10^{-4}$ und $10^{-9}$ molaren Konzentration betragen. Der Dosisbereich in-vivo kann zwischen 0,01 und 50 mg/kg/Tag, vorzugsweise zwischen 0,05 und 20 mg/kg/Tag betragen.

Die präsynaptischen Dopamin-Rezeptor regulierenden (z.B. agonistische) Eigenschaften der erfindungsgemässen Verbindungen lassen sich in-vitro im Dopamin-Bindung-Assay nach Standardmethoden durch Ersatz des Dopaminagonisten 2-Amino-6,7-dihydroxy-1,2,3,4-tetrahydronaphthaline ($^3$H-ADTN) aus Membranen von Nucleus candatus von Kälbern bestimmen.

In der Bindung-Assay, werden dreifach augefertigte 2 ml Proben (aequivalent zu 5 mg/ml Gehirngewebe) aus Membranen vom Nucleus candatus von Kälber-Gehirn Suspension 60 Minuten bei 25°C mit 0,2 nM $^3$H-ADTN in oder ohne Gegenwart von verschiedenen Konzentrationen der erfindungsgemässen Verbindungen inkubiert. Die Reaktion wird durch das Filtrieren mit 5 ml von kaltem 50 mM Tris-HCl Puffer pH 7,7 beendet. Die Filtrate werden in Scintillationsvials mit 5 ml Scintillationslösung gefüllt, durch 90 Minuten dauerndes starkes mechanisches Schütteln getrennt und die Radioaktivität wird gemessen.

Die $IC_{50}$-Wert (Konzentrationen an Wirkstoff, welche 50 %-ige Hemmung der spezifischen Bindung von 0,2 nM $^3$H-ADTN nötig sind) werden graphisch bestimmt.

Die in-vivo Wirkung als präsynaptischer Dopaminagonist (auch als Dopamin-Autorezeptor-Agonsit Wirkung genannt) lässt sich in Gamma-Butyrolacton (GBL)-Modell an der Ratte anhand der von Walters und Roth in Naunyn-Schmiederberg's Arch. Pharmacol. 296, 5 (1976) beschriebenen Methode bestimmen. In diesem pharmazeutischen Modell inkubiert der präsynaptische Dopamin-Agonist die GBL-induzierte Anreicherung der Dopamin-Vorstufe DOPA im Gehirn nach vorheriger Behandlung mit 3-Hydroxy-benzylhydrazin

(NDS-1015), einen DOPA-Decarboxylase-Inhibitor.

Die Test-Verbindungen werden in 0,9%iger Kochsalzlösung, wenn nötig mit Zugabe von Natriummetabisulfit zur Verhinderung von Oxidation, gelöst. Man verabreicht den Versuchstieren i.p. Injektionen von Lösungen der Testsubstanzen oder Kochsalzlösung, nachfolgend bei Gamma-Butyrolacton (GBL, 750 mg/kg i.p.) oder bei den Kontrolltieren mit Kochsalzlösung 15 Minuten später, und dann NSD-1015 (100 mg/kg i.p.) 5 Minuten nach der Verabreichung von GBL. 30 Minuten nach der Verabreichung von NSD-1015 werden die Versuchstiere getötet, Hirne herausgenommen, Striatum isoliert und bei -70°C tiefgefroren bis zur Deproteinization, extrahiert und analysiert. Die $ED_{50}$-Werte (Dosis bei welcher die DOPA-Anreicherung zu 50 % inhibiert ist) werden durch Aufzeichnung von prozentaulfer Hemmung von DOPA-Akkumulation gegen $Log_{10}$ der Dosis einer Testverbinduing bestimmt. Die gleiche Methode ist auch für die orale Verabreichung verwendbar.

Die Selektivität der erfindungsgemässen Verbindungen als präsynaptische Dopamin-Rezeptor-Agonisten wird in-vitro durch Bindungsstudien der Verdrängung von $^3$H-Spiroperidol aus post-synaptischen Dopamin-Rezeptoren bestimmt. Schwache Bindung in diesem Test ist indikativ für die Selektivität.

In-vivo lässt sich diese Selektivität durch Messen der Aufhebungsgrades der mit Reserpin induzierten Hypomobilität bei der Ratte bestimmen, beschrieben in Life Sciences 28, 1225 (1981). Das relative Ausbleiben einer solchen Aufhebung bei effektiver Dosis (z.B. in GBL-Modell) ist Anzeige der selektiven präsynaptischen Dopamin-Rezeptor-Agonist Wirkung.

So hat beispielsweise die erfindungsgemäss Verbindung 5-Dipropylamino-5,6-dihydro-4H-thieno[2,3-b]-thiopyran-hydrochlorid einen $IC_{50}$-Wert von $1,5 \times 10^{-7}$ M im $^3$H-ADTN präsynaptischen Dopamin-Rezeptor-Assay.

Die erfindungsgemässe Verbindung 5-Dipropylamino-5,6-dihydro-4H-thieno[2,3-b]thiopyran zeigt einen $ED_{50}$-Wert von 2,6 mg/kg i.p. und 5,6 mg/kg p.o. in dem GBL-Modell bei der Ratte.

Die besagten vorteilhaften Eigenschaften belegen die nützliche therapeutische Verwendbarkeit der erfindungsgemässen Verbindungen in Säugern (inkl. Menschen) als Arzneimittel mit Wirkung auf das zentrale Nervensystem. Die erfindungsgemässen Verbindungen können als selektive präsynaptische Dopamin-Rezeptor-Modulatoren zur Behandlung von Störungen des zentralen Nervensystems, z.B. als Neuroleptika oder Antipsychotika, verwendet werden.

Die erfindungsgemässen Verbindungen können nach folgenden Verfahren hergestellt werden:

a) Kondensation einer Verbindung der Formel II

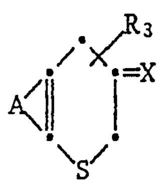

(II),

worin A und $R_3$ die vorn genannten Bedeutungen haben, X Sauerstoff oder reaktives verestertes Hydroxy zusammen mit Wasserstoff bedeutet, oder ein Mono-oder Di-S-Oxid einer Verbindung der Formel II, mit einer Verbindung der Formel III

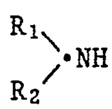

(III),

worin $R_1$ und $R_2$ die vorn genannten Bedeutungen haben; oder

b) Alkylierung einer Verbindung der Formel IV

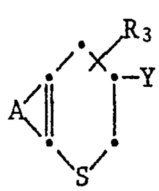

(IV),

worin A und $R_3$ die vorn genannten Bedeutungen haben, und Y $-NH_2$, $NHR_1$ oder $NHR_2$ bedeutet, oder ein Mono-oder Di-S-Oxid einer Verbindung der Formel IV mit einem rekationsfähigen Ester eines Alkohols der

Formel V

$R_1$-OH oder $R_2$-OH (V),

worin $R_1$ und $R_2$ die vorn genannten Bedeutungen haben, oder mit einem $R_1$-OH und $R_2$-OH entsprechenden Aldehyd unter Einhaltung von reduzierenden Bedingungen; oder

c) Reduktion einer Verbindung der Formel VI

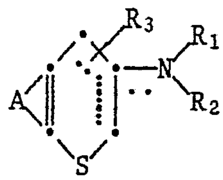

(VI),

worin $R_1$, $R_2$, $R_3$ und A die obengenannten Bedeutungen haben, und die punktierten Bindungslinien eine Doppelbindung in einer der angezeigten Positionen darstellen, oder ein Mono-oder Di-S-Oxid einer Verbindung der Formel VI; oder

d) Reduktion einer Verbindung der Formel VII

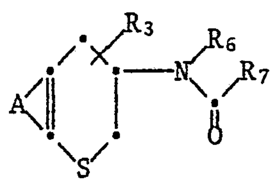

(VII),

worin $R_6$ $R_1$ oder $R_2$ bedeutet, A und $R_3$ die vorn genannten Bedeutungen haben; und $R_7$ Niederalkyl mit 1 - 6 C-Atomen, Arylniederalkyl mit 1 - 6 C-Atomen oder Wasserstoff bedeutet; oder ein Mono-oder Di-S-Oxid einer Verbindung der Formel VII; oder

e) Umwandlung eines Derivates, wie z.B. Azid-, primäres Amin-, N-Hydroxyamin-oder N-Acyloxyamin-Derivat, einer Carbonylsäure der Formel VIII

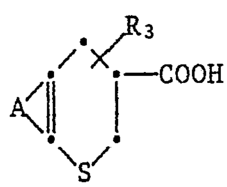

(VIII),

worin A und $R_3$ die vorn genannten Bedeutungen haben, oder eines mono-S-oder Di-S-Oxids einer Verbindung der Formel VIII, und eine Verbindung der Formel I, worin $R_1$ und $R_2$ Wasserstoff bedeuten isoliert; und gewünschtenfalls reaktive funktionelle Gruppen intermediär bei der Ausführung eines der genannten Verfahren schützt und anschliessend die freie Verbindung der Formel I isoliert und/oder eine erhaltene Verbindung der Formel I in eine andere Verbindung der Formel I umwandelt, und/oder eine erhaltene freie Verbindung in ein Salz oder ein Salz in die freie Verbindung oder in ein anderes Salz umwandelt, und/oder ein erhaltenes Gemisch von Isomeren oder Racematen in einzelne Isomere oder Racemate auftrennt, und/oder ein Racemat in die optischen Antipoden aufspaltet.

Eine reaktionsfähige, veresterte Hydroxygruppe in den genannten Verfahrensvarianten ist beispielsweise eine Hydroxygruppe, welche durch eine starke Säure, insbesondere eine starke anorganische Säure, wie Halogenwasserstoff, z.B. Chlor-, Brom-oder Jodwasserstoff, oder Schwefelsäure, oder durch eine starke organische Säure, beispielsweise eine Sulfonsäure, wie eine aliphatische oder aromatische Sulfonsäure, z.B. Methansulfonsäure, p-Toluolsulfonsäure oder 4-Bromobenzolsulfonsäure, verestert ist. Eine solche reaktions fähige, veresterte Hydroxygruppe ist insbesondere Halogen, z.B. Chlor, Brom oder Jod, oder durch aliphatische oder aromatische Gruppen substituiertes Sulfonyloxy, z.B. Mesyloxy oder Tosyloxy.

Die in den Ausgangsmaterialien, Zwischenstufen und in Verbindungen der Formel I vorhandenen funktionellen Gruppen, insbesondere die Carboxy-, Amino-, Hydroxy-und Sulfogruppen, sind gegebenenfalls durch solche Schutzgruppen (conventional protecting groups) geschützt, die üblicherweise in der Penicillin-, Cephalosporin-und Peptidchemie verwendet weden. Diese Schutzgruppen sollen die betreffenden funktionellen Gruppen gegen unerwünschte Nebenreaktionen, wie Acylierungen, Verätherungen, Veresterungen

Oxydationen, Solvolyse etc., schützen und unter schonenden Reaktionsbedingungen leicht abspaltbar sein, das heisst unter Vermeidung von Spaltung, z.B. solvolytischer, reduktiver, photolytischer oder auch enzymatischer Spaltung, sowie anderer Nebenreaktionen.

Der Schutz von funktionellen Gruppen mit solchen Schutzgruppen, die Schutzgruppen selbst, sowie ihre Abspaltungsreaktionen, sind beispielsweise in "Protective Groups in Organic Chemistry", Plenum Press, London und New York 1973, in Greene, Th.W. "Protective Groups in Organic Synthesis", Wiley, New York 1981, in "The Peptides", Vol. I. Schröder and Lubke, Academic Press, London und New York 1965, sowie in "Methoden der organischen Chemie", Houben-Weyl, 4. Auflage, Bd. 15/I Georg Thieme Verlag, Stuttgart 1974, beschrieben.

Die Herstellung von Verbindungen der Formel I gemäss Verfahrensvariante a) kann man analog bekannten N-Alkylierungsreaktionen durchführen.

Die Herstellung von Verbindungen der Formel I durch reduktive N-Alkylierung von Ausgangsmaterialien, worin X Oxo bedeutet, kann man analog bekannten Reaktionsbedingungen, z.B. mit Hydridreduktionsmitteln, wie Natriumcyanborhydrid, durchführen. Die reduktive Aminierung mit Hydridreduktionsmitteln wird vorzugsweise in inertem Lösungsmittel, wie Methanol oder Acetonitril, bevorzugt in Gegenwart einr Säure, wie Chlorwasserstoffsäure oder Essigsäure, durchgeführt.

Die Herstellung von Verbindungen der Formel I gemäss Verfahrensvariante a) aus Ausgangsmaterialien, worin X eine reaktionsfähige, veresterte Hydroxygruppe und gleichzeitig Waserstoff darstellt, führt man gegebenenfalls in Gegenwart von Basen, wie Triethylamin oder Kaliumcarbonat, in einem inerten Lösungsmittel analog bekannten Reaktionsbedingungen für N-Alkylierungen durch.

Die Herstellung von Verbindungen der Formel I gemäss Verfahrensvariante b) wird unter den für die Verfahrensvariante a) genannten Reaktionsbedingungen durchgeführt. beispielsweise bei N-Alkylierungen, wenn das Ausgangsmaterial ein reaktiver Ester eines Alkohols der Formel $R_1$-OH oder $R_2$-OH ist, oder N-Alkylierungen unter reduzierenden Bedingungen, wenn das Ausgangsmaterial ein Aldehyd, z.B. in Niederalkanal oder ein Arylniederalkanal, ist.

Die Herstellung von Verbindungen der Formel I gemäs Verfahrensvariante c) führt man unter solchen Reaktionsbedingungen durch, welche für Reaktionen, in den Kohlenstoff-Stickstoff-Doppelbindungen oder Kohlenstoff-Kohlenstoff-Doppelbindungen in einer Enamin-Gruppierung gesättigt werden, bekannt sind, z.B. durch Umsetzung mit chemischen Reduktionsmitteln wie Natriumcyanoborhydrid, unter den Bedingungen, die für diese Art bekannt sind, z.B. bei Raumtemperatur oder unter Erwärmen, in einem polaren Lösungsmittel, wie z.B. Isopropanol.

Die Herstellung von Verbindungen der Formel I gemäss Verfahrensvariante d) führt man nach bekannten Verfahren für Reduktion einer Aminogruppe, z.B. durch Reduktion mit Hydridreduktionsmitteln, wie Lithiumaluminiumhydrid, oder einem Boran oder Diboran, in inerten Lösungsmitteln, wie Tetrahydrofuran oder Diethylether, vorzugsweise bei Raumtemperatur oder unter Erwärmen, durch.

Die Herstellung von Verbindungen der Formel I gemäss Verfahrensvariante e) wird nach bekannten Verfahren durchgeführt, wie z.B. durch Hofmann'sche Umwandlung der Amine, Umwandlung der Acylazide nach Curtius, Umwandlung der N-Acyloxyamine nach Lossen und durch Umsetzung der Carboxylgruppe mit Hydrazinhydrat nach Schmidt.

Die Ketone der Ausgangsverbindungen der Formel II, gegebenenfalls substituierte 5,6-Dihydro-4H-thieno[2,3-b]thiopyran-5-one und 6,7-Dihydro-5H-thieno[3,2-b]thiopyran-6-one, können insbesondere durch Kondensation, z.B. einer gegebenenfalls substituierten Thiophen-2-, oder 3-Thiol-Verbindung mit Alphahalomethylacrylsäure mit nachfolgender Cyclisierung der entstandenen Alpha-thiophenthiomethyl-acrylsäure unter Erwärmen hergestellt werden, dabei erhält man die gegebenenfalls substituierte 5,6-Dihydro-4H-thieno-[2,3-b]-thiopyran-5-carbonsäure oder 6,7-Dihydro-5H-thieno[3,2-b]thiopyran-6-carbonsäure (Formel VIII).

Durch Dehydrierung, z.B. durch Behandlung mit N-Chlorosuccinimid, erhält man die entsprechende 4-H-Thieno[2,3-b]thiopyran-5-carbonsäure oder 5H-Thieno[3,2-b]thiopyran-6-carbonsäure. Mit Degradierung zum Amin, z.B via Azid, und durch saure Hydrolyse erhält man den entsprechenden 5,6-Dihydro-4H-thieno-[2,3-]thiopyran-5-on oder 6,7-Dihydro-5H-thieno[3,2-b]thiopyran-6-on.

Ein gegebenenfalls substituierter 4H-Thieno-[2,3-b]thiopyran oder 5H-Thieno[3,2-b]-thiopyran wird beispielsweise mit eine Halogenierungsmittel, z.B. N-Bromosuccinimid oder N-Bromoacetamid in einem Hydroxylgruppen-haltigen Lösungsmittel, wie in wässrigem Aceton, umgesetzt, und durch Umsetzung mit einer nicht-wässerigen Base, z.B. Natriumhydrid in Tetrahydrofuran, erhält man das entsprechend substituierte Epoxid. Nach Umlagerung mit einem Säure-haltigen Reagens, z.B. Zinkjodid in Toluol, erhält man die entsprechenden Ketone der Formel II.

Die primären und sekundären 5-Amino-5,6-dihydro-4H-thieno[2,3-b]-thiopyrane und 6-Amino-6,7-dihydro-5H-thieno[3,2-b]thiopyrane der Formel IV, für Verfahrensvariante b) können aus den Ausgangsstoffen der Formel II, worin X Oxo bedeutet, durch Umsetzung mit Ammoniak, einem primären Amin der Formel $R_1NH_2$ oder $R_2NH_2$ vorzugsweise mit einem Säureadditionsalz davon, in Gegenwart eines Reduktionsmittels, vorzugsweise Natriumcyanoborydrid, hergestellt werden.

Die primären Amine der Formel IV, welche für die Verfahrens-variante b) verwendbar sind, können ebenfalls durch Curtius-Abbau von einer entsprechenden 5,6-Dihydro-4H-thieno[2,3-b]thiopyran-5-carbonsäure oder 6,7-Dihydro-5H-thieno[3,2-b]thiopyran-6-carbonsäure (die Herstellung von diesen Verbindungen ist weiter vorn beschrieben), z.B. mit Diphenylphosphorylazid oder wie bei der Verfahrensvariante e) und in den Beispielen beschrieben ist, hergestellt werden.

Die Zwischenprodukte der Formel VI können durch Umsetzung von entsprechend substituierten Ketonen der Formel II, worin X Oxo bedeutet, mit einem Amin der Formel III unter Reaktionsbedingungen, welche durch Wasserentzug gekennzeichnet sind, z.B. in Gegenwart von Molekularsieb, Bortrifluoridetherat oder p-Toluolsulfonsäure, in einem inerten Lösungsmittel, wie Toluol oder Methylenchlorid, hergestellt werden.

Die Zwischenprodukte der Formel VII können durch Acylierung einer Verbindung der Formel IV, worin Y $NHR_1$ oder $NHR_2$ darstellt, mit einer Carbonsäure der Formel $R_7$-COOH, worin $R_1$, $R_2$ und $R_7$ die vorn genannten Bedeutungen haben, in Gegenwart eines Kondensationsmittels, wie Dicyclohexylcarbodiimid, oder mit einem reaktionsfähigen, funktionellen Derivat davon, z.B. Acylhalid, wie Säurechlorid, oder einem gemischten Anhydrid, z.B. von einem Niederalkylhalocarbonat, swie Chlorameisensäureethylester, in Gegenwart von einem inerten Lösungsmittel, wie Methylenchlorid, und einer Base, wie Pyridin, hergestellt werden.

Die Zwischenprodukte der Formel VIII (auch Zwischenprodukte für die Herstellung von Verbindungen der Formel II), welche in der Verfahrensvariante e) zur Anwandung gelangen, können analog wie oben beschrieben oder wie in den Beispielen dargestellt ist hergestellt werden und dann zu den entsprechenden primären Aminen, Aziden oder N-Acyloxyaminen nach bekannten Methoden umgewandelt werden, wie z.B. zum Azid mit Diphenylphosphorylazid, zum N-Acyloxyamin durch Umwandlung der Carbonsäure in einem Niederalkylester und dann in die Hydroxamsäure, welche dann, z.B. mit einem Säurechlorid, acyliert wird.

Erfindungsgemässe Verbindungen, die nach irgendeiner der weiter vorn beschriebenen Verfahrensvarianten erhalten werden, können analog bekannten Verfahren, wie zum Beispiel im folgenden Abschnitt erläutert wird, in andere erfindungsgemässe Verbindungen der Formel I umgewandelt werden.

So können beispielsweise primäre Amine in sekundäre Amine oder sekundäre Amine in tertiäre Amine nach bekannten Methoden umgewandelt werden.

Die Verbindungen der Formel I oder Zwischenprodukte können z.B. in die entsprechenden Mono-oder Di-S-Oxide umgewandelt werden, z.B. durch Behandlung mit Persäure, insbesondere mit m-Chlorperbenzoesäure, erhält man mono-S-Oxide oder Di-S-Oxide entsprechend der verwendeten Menge von Persäure. Die Sulfoxide könen auch durch Behandlung mit einem Salz der Perjodsäure, wie Natriumperjodat, hergestellt werden.

Die Verbindungen der Formel Ia oder Ib, worin $R_4$ Wasserstoff bedeutet und $R_1$ und $R_2$ von Wasserstoff verschiedene Bedeutung haben, können in entsprechenden Verbindungen, worin $R_4$ Niederalkyl bedeutet, durch Behandlung mit einer starken Base, wie Butyllithium, in einem inerten Lösungsmittel, wie Tetrahydrofuran, und durch Behandlung mit einem Niederalkylhalide umgewandelt werden.

Die oben genannten nachträglichen Reaktionen werden nach an sich bekannten Methoden, in Gegenwart oder Abwesenheit von Verdünungsmitteln, vorzugsweise solchen, welche gegenüber den Reagenzien inert sind und diese lösen, Katalysatoren, Kondensations-oder anderen oben genannten Mitteln und/oder in einer inerten Atmosphäre, unter Kühlung, bei Zimmertemperatur oder bei erhöhter Temperatur, vorzugsweise beim Siedepunkt des verwendete Lösungsmittels bei normalem oder erhöhtem Druck, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird. Immer wenn gewünscht, werden die oben genannten Verfahren erst durchgeführt, nachdem alle potentiell störenden, reaktionsfähigen funktionellen Gruppen in geeigneter Weise geschützt sind, z.B. wie oben und in den Beispielen illustriert.

In den genannten Reaktionen werden vorteilhafterweise solche Ausgangsstoffe verwendet, welche zu den weiter vorn als besonders bevorzugt beschriebenen Verbindungn führen.

Die Erfindung betrifft auch neue Ausgangsstoffe und Verfahren zu ihrer Herstellung.

Abhängig von den gewählten Ausgangsstoffen und Methoden können die neuen Verbindungen in Form von Isomeren oder als Isomerengemische vorliegen, zum Beispiel als geometrische Isomeren oder Mischungen davon (vis oder trans), als optische reine Antipoden, Racemate oder Diastereomeren.

Erhaltene geometrische oder diastereomere Isomerengemische der obigen Verbindungen oder Zwischenproudkte können nach an sich bekannten Methoden in die einzelnen racemischen oder optisch aktiven Isomeren getrennt werden, z.B. durch fraktionierte Destillation, Kristallisation und/oder Chromatographie. Racemische Produkte können gleichfalls in die optischen Antipoden gespalten werden, z.B. durch Trennung ihrer diastereomeren Salze, wie gemäss J. Org. Chem. 43, 3803 (1978), z.B. durch faktionierte Kristallisation von d-und ℓ-(Tartraten, Mandelaten, Camphersulfaten oder Dibenzoyltartraten) Salzen.

Bevorzugt wird jeweils das wirksamere Isomere und/oder der wirksamere Antipode der erfindungsgemässen Verbindungen isoliert.

Die Verbindungen der vorliegenden Erfindung werden entweder in freier oder in Form ihrer Salze erhalten. Jede erhaltene Base kann in das entsprechende Säureadditionssalz umgewandelt werden, vorzugsweise unter Verwendung einer therapeutisch annehmbaren Säure oder eines Anionenaustauschers. Erhaltene Salze können in die entsprechenden freien Basen umgewandelt werden, zum Beispiel unter Verwendung einer stärkeren Base, beispielsweise eines Metall-oder Ammoniumhydroxids oder eines basischen Salzes, z.B. eines Alkalimetallhydroxids oder -carbonats, oder eines Kationenaustauschers. Diese oder andere Salze, etwa Pikrate, können auch zur Reinigung der erhaltenen Basen verwendet werden, indem man die Basen in Salze überführt, diese abtrennt und reinigt, und aus den Salzen wiederum die Base freisetzt. Infolge der engen Beziehung zwischen den freien Verbindungen und ihren Salzen sind in diesem Zusammenhang unter freien Verbindungen sinn-und zweckmässig gegebenenfalls immer auch die entsprechenden Salze zu verstehen.

Die Verbindungen und ihre Salze können auch in Form ihrer Hydrate erhalten werden, oder sie können andere zur Kristallisation verwendete Lösungsmittel einschliessen.

Die vorliegende Erfindung betrifft ebenfalls die Verwendung von Verbindungen der Formel I zur Herstellung von pharmazeutischen Zusammensetzungen, insbesondere pharmazeutischen Zusammensetzungen mit stimulierender Wirkung auf präsynaptische Dopaminrezeptoren, welche eine wirksame Menge einer erfindungsgemässen Verbindung und in Kombination mit einem oder mehreren pharmazeutisch annehmbaren Trägermaterialien enthalten.

Die pharmakologisch aktiven Verbindungen der Erfindung sind bei der Herstellung von pharmazeutischen Zusammensetzungen verwendbar, die eine wirksame Menge derselben in Verbindung oder Beimischung mit Excipienten oder Trägern, die für die enterale, transdermale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium-oder Calciumsalz und/oder Polyethylenglykol, für Tabletten auch c) Bindemitteln, z.B. Magnesiumaluminiumsilikat, Stärkepaste, Gelatine Tragant, Methylcellulose, Natriumcarboxymethylcellulose und/oder Polyvinylpyrrolidon, gewünschtenfalls d) Zerteilungs-bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvanzien, wie Konservierungs-, Stabilisierungs-, Netz-oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier-bzw. Ueberzugsmethoden hergestellt und enthalten etwa 0,1 bis 75 %, vorzugsweise etwa 1 bis 50 %, des aktiven Bestandteils.

Geeignete Formulierungen für die transdermale Anwendung enthalten eine wirksame Menge einer Verbindung der Formel I zusammen mit Trägermaterial. Geeignete Trägermaterialien enthalten pharmazeutisch annehmbare Hilfsstoffe, welche den Durchgang durch die Haut ermöglichen. Insbesondere haben transdermale therapeutische Systeme die Form eines Pflasters mit einer Schutzschicht, einem Wirkstoffreservoir, gegebenenfalls mit Trägermaterialien, und einer die Abgabe bestimmenden Kontrollschicht, durch welche der Wirkstoff auf die Haut mit kontrollierter und bestimmbarer Geschwindigkeit über einen längeren Zeitabschnitt abgegeben wird. Das System hat gegebenenfalls noch eine Klebschicht.

Die vorliegende Erfindung betrifft ebenfalls die Verwendung von Verbindungen der Formel I mit stimulierenden Eigenschaften auf präsynaptische Dopaminrezeptoren und von pharmazeutischen Zusammensetzungen mit den erfindungsgemässen Verbindungen zur Behandlung von Säugern inkl. Menschen bei Störungen des zentralen Nervensystems als Folge von Stimulierungserscheinungen von präsynaptischen Dopaminrezeptoren, wie Dyskinesie, Parkinsonismus und insbesondere von psychotischen Zuständen, z.B. Schizophrenie.

Die applizierbare Wirkstoffdosis ist speziesabhängig sowie vom Körpergewicht, dem Alter und individuellen Zustand und der Darreichungsform abhängig.

Die Einheitsdosis für einen Säuger, z.B. Mensch, von ca. 50 bis 70 kg Gewicht beträgt zwischen 10 und 100 mg des Wirkstoffs.

Die folgenden Beispiele sollen die Erfindung erläutern und keine Einschränkungen darstellen. Die Temperaturen sind in Celsiusgraden angegeben, und sämtliche Teile sind in Form von Gewichtsteilen angegeben. Wenn nicht anders erwähnt, werden sämtliche Verdamfungen unter vermindertem Druck vorzugsweise zwischen etwa 15 und 100 mg Hg durchgeführt.

Beispiel 1

a) Ein Gemisch von 10,0 g 5,6-Dihydro-4H-thieno[2,3-b]thiopyran-5-(N-benzoyloxy)carboxamid und 195 ml Ethanollösung von 0,165 molaren Natriumethoxid wird unter Rückflussbedingungen während 3 Stunden erhitzt und danach filtriert. Das Filtrat wird bis zur Trockenheit eingedampft und der Rückstand mit 60 ml Aether extrahiert. Das Extrakt wird mit 50 ml 10 %-iger Natriumhydroxidlösung gewaschen, und dann mit Ammoniumchloridlösung saturiert, über Magnesiumsulfat getrocknet und das Lösungsmittel abgedampft. Man erhält 5-Ethoxycarbonylamino-5,6-dihydro-4H-thieno[2,3-b]thiopyran als Oel.

Ein Gemisch von 5,3 g 5-Ethoxycarbonylamino-5,6-dihydro-4H-thieno[2,3-b]thiopyran, 20 ml 26 %iger wässriger Natriumhydroxidlösung und 20 ml 1,2-Propandiol wird bei 140° während 3 Stunden erhitzt. Das Reaktionsgemisch wird abgekühlt und vorsichtig mit 29 ml 6 N Salzsäure bis zur Gasentwicklung angesäuert. Das erhaltene Gemisch wird mit 50 %-iger Natriumhydroxidlösung versetzt und mit Ethylacetat extrahiert. Das Extrakt wird zur Trockenheit eingedampft, der Rückstand in Aether gelöst, die Aetherlösung mit Wasser gewaschen und zur Trockenheit eingedampft. Der Rückstand wird in 40 ml Ethylacetat gelöst und 41 ml Chlorwasserstofflösung in Ethylacetat (0,535 molar) zugefügt. Nach dem Trocknen erhält man 5,6-Dihydro-4H-thieno[2,3-b]thiopyran-5-amin-hydrochlorid.

Das Ausgangsmaterial wird folgendermassen erhalten:

Eine Lösung von 16,8 mg Thiophen in 50 ml Tetrahydrofuran wird auf -10° gekühlt und eine 0,2 molare Lösung von n-Butyllithium in Hexan unter ständiger Kühlung auf -10° (unter Stickstoffatomosphäre) zugefügt, so dass die Temperatur ungefähr bei -5° bleibt. Das Reaktionsgemisch wird während 30 Minuten bei Temperaturen zwischen -10° und -5° gerührt, dann 6,4 g granulierter Schwefel langsam zugefügt und das Reaktionsgemisch noch 2 Stunden bei -5° bis 0° gerührt. Eine kalte Lösung von 33,0 g alpha-Brommethylacrylsäure gelöst bei 0° in 8,3 g Natriumhydroxidlösung in 125 ml Wasser wird langsam unter Kühlung, so dass die Temperatur bei 10° bleibt, zugefügt. Nach einer Stunde Reaktionszeit bei 0° bis 5° und unter Rühren wird das Reaktionsgemisch während 2 Stunden langsam auf 20° erwärmt, zweimal mit 25 ml Toluol extrahiert und die Toluolextrakte werden einmal mit 25 ml gesättigter Natriumchloridlösung saturiert.

Die wässrige Phase und die wässrigen Waschanteile werden zusammengefügt, mit Aktivkohle behandelt und durch Hyflo filtriert. Die basische wässrige Lösung wird dann mit 150 - 250 ml Wasser verdünnt, mit 40 ml 6 N HCl bis zu pH 1,0 - 2,0 behandelt und während 30 Minuten bei 20° gerüht. Das Produkt wird gesammelt, mit Wasser gewaschen und getrocknet. Man erhält alpha-[(2-Thienyl)-thiomethyl]-acrylsäure; Silicagel: $R_f$ = 0,75, Eluationsmittel: Toluen/Ethylacetat/Essigsäure (70 : 30 : 10).

Eine Lösung von 95,0 g alpha-[(2-Thienyl)-thiomethyl]-acrylsäure in 190 ml Dimethylformamid wird während 40 Minuten bei 135° erhitzt und bei 100° unter reduziertem Druck auf ein Volumen von 135 ml abgedampft. Der Rückstand wird mit 400 ml t-Butylmethyläther gelöst, 81,3 g Dicyclohexylamin zugegeben und das Reaktionsgemisch über Nacht bei Raumtemperatur gerührt. Das auskristallisierte Produkt wird gesammelt, mit t-Butylmethyläther gewaschen und getrocknet. Man erhält 5,6-Dihydro-4H-thieno[2,3-b]-thiopyran-5-carbonsäure Dicyclohexylammoniumsalz; Silicagel: $R_f$ = 0,63, Eluationsmittel: Toluen/Ethylacetat/Essigsäure (70 : 30 : 10).

Zu einem Gemisch von 25,0 g 5,6-Dihydro-4H-thieno[2,3-b]thiopyran-5-carbonsäure Dicyclohexylammoniumsalz, 100 ml Methanol und 100 ml Toluol wird 6,84 g konz. Schwefelsäure zugefügt. Das Reaktionsgemisch wird während 4 1/2 Stunden unter Rückfluss erhitzt und im Vakuum konzentriert. 50 ml Ethylacetat und 50 ml Wasser werden zugefügt. Die Ethylacetat-Schicht wird separiert und die wässrige Schicht mit 45 ml Ethylacetat extrahiert. Die kombinierten Ethylacetatextrakte werden zuerst mit verdünnter Schwefelsäure, dann mit verdünntem Ammoniumhydroxid, dann mit Natriumchloridlösung und mit Wasser gewaschen, getrocknet und bis zur Trockenheit eingedampft; man erhält 5,6-Dihydro-4H-thieno-[2,3-b]-thiopyran-5-carbonsäuremethylester, der durch Destillation gereinigt wird.

Einer Lösung von 8,7 g granuliertem Kaliumhydroxid in 25 ml absolutem Methanol wird eine Lösung von 5,9 g Hydroxylaminhydrochlorid in 35 ml absolutem Methanol zugefügt. Zu diesem Gemisch wird unter

Stickstoffatmosphäre 9,1 g 5,6-Dihydro-4H-thieno2,3-b]thiopyran-5-carbonsäuremethylester zugefügt. Nach einer Stunde wird das Gemisch zur Trockenheit eingedampft. 36 ml Wasser und 36 ml Ethylacetat werden zugefügt, das Gemisch wird bei Raumtemperatur gerührt, mit 5,5 ml konzentrierter Chlorwasserstoffsäure 10 bis 15 Minuten angesäuert. Die Ethylacetat-Schicht wird abgetrennt, die wässrige Schicht mit Ethylacetat extrahiert und die kombinierten Ethylacetatextrakte werden zu Trockenheit eingedampft. Der Rest wird aus Methylenchlorid kristallisiert, man erhält 5,6-Dihydro-4H-thieno-[2,3-b]thiopyran-5-(N-hydroxy)-carboxamid.

Eine Lösung von 9,79 g Benzoylchlorid in 45 ml Methylenchlorid wird tropfenweise zu einer Lösung von 15,0 g 5,6-Dihydro-4H-thieno[2,3-b]thiopyran-5-(N-hydroxy)carboxamid und 7,05 g Triethylamin in 105 ml Methylenchlorid so zugegeben, dass die Temperatur zwischen 0° und 5° gehalten wird. Es ist gestattet, das Reaktionsgemisch bis auf 10° zu erwärmen und 150 ml Wasser wird in 4 Portionen zugefügt. Das erhaltene Produkt wird gesammelt und getrocknet, man erhält 5,6-Dihydro-4H-thieno[2,3-b]thiopyran-5-(N-benzoyloxy)-carboxamid.

b) Wenn 3-Thiophenthiol als Ausgangsmaterial genommen wird, erhält man 6,7-Dihydro-5H-thieno[3,2-b]thiopyran-6-amin.

Beispiel: 2

a) 700 ml 50 %-iger Natriumhydroxidlösung wird portionsweise zu einem Gemisch von 114,65 g 5,6-Dihydro-4H-thieno[2,3-b]thiopyran-5-amin-hydrochlorid, 940 g n-Propyljodid und 18,3 g tetra-n-Butylammoniumbromid in 700 ml Toluol zugegeben. Das Reaktionsgemisch wird unter Rückfluss 20 Stunden auf 100° erhitzt. Die organische Schicht wird getrennt und die wässrige Phase mit 300 ml Toluol extrahiert. Die kombinierten Toluolextrakte werden mit Wasser gewaschen, getrocknet und das Lösungsmittel im Vakuum entfernt. Der Rückstand wird dann im Hochvakuum destilliert und man erhält N,N-Dipyropyl-5,6-dihydro-4H-thieno[2,3-b]thiopyran-5-amin, mit einem Siedepunkt von 155° (0,5 mm Hg).

Eine Lösung von 4,0 g N,N-Dipropyl-5,6-dihydro-4H-thieno[2,3-b]thiopyran-5-amin in 20 ml t-Butylmethyläther wird langsam unter Rühren zu einem Gemisch von 5,42 g 2,4 M ethanolischer Chlorwasserstofflösung in 45 ml t-Butylmethyläther zugefügt. Das auskristallisierte Produkt wird abgetrennt, mit dem Lösungsmittel gewaschen und getrocknet; man erhält N,N-Dipropyl-5,6-dihydro-4H-thieno[2,3-b]thiopyran-5-amino-hydrochlorid mit einem Schmelzpunkt von 163 - 167°.

In analoger Weise werden hergestellt:

b) N,N-Dibutyl-5,6-dihydro-4H-thieno[2,3-b]thiopyran-5-amin-hydrochlorid, F.p. 161 - 164°;

c) N,N-Diethyl-5,6-dihydro-4H-thieno-[2,3-b]thiopyran-5-amin;

d) N,N-Dipropyl-6,7-dihyro-5H-thieno[3,2-b]thiopyran-6-amin.

Beispiel 3:

Ein Gemisch von 9,15 g 5,6-Dihydro-4H-thieno[2,3-b]thiopyran-5-carbonsäure, 10 ml Diphenylphosphorylazid und 6,5 ml Triethylamin in 200 ml t-Butanol wird während 5 Stunden unter Rückfluss erhitzt. Nach Abzug von Lösungsmittel im Vakuum wird der Rückstand in Aether gelöst und mit 1 N Natriumhydroxid gewaschen. Nach dem Trocknen über Magnesiumsulfat wird das Lösungsmittel im Vakuum entfernt; man erhält N-t-Butoxycarbonyl-5,6-dihydro-4H-thieno[2,3-b]-thiopyran-5-amin und N-t-Butoxycarbonyl-2-t-butyl-5,6-dihydro-4H-thieno[2,3-b]thiopyran-5 -amin. Das Gemisch wird mit 25 ml Trifluoressigsäure versetzt und 15 Minuten unter Rückfluss erhitzt. Nach Abziehen des Lösungsmittels wird der Rückstand mit 40 ml N-Propyljodid in 100 Toluol in Gegenwart von 27 g Natriumcarbonat in 150 ml Wasser behandelt. Das erhaltene Gemisch wird 3 Tage unter Rührung beim Rückfluss erhitzt. Das Lösungsmittel wird entfernt und die organische Schicht an Silicagel chromatographiert; man erhält 2-t-ButylN,N-dipropyl-5,6-dihydro-4H-thieno[2,3-b]thiopyran-5-amin-hydrochlorid, F.p. 227 - 233°, und N,N-Dipropyl-5,6-dihydro-4H-thieno-[2,3-b]-thiopyran-5-amin-hydrochlorid, F.p. 163 - 167° (Beispiel 2).

Beispiel 4:

Ein Gemisch von 8,31 g 5,6-Dihydro-4H-thieno[2,3-b]thiopyran-5-amin-hydrochlorid, 17,4 ml Ethyldiisopropylamin, 4,07 g Propionylchlorid in 80 ml Methylenchlorid wird während 30 Minuten bei Raumtemperatur gehalten. Das Reaktionsgemisch wird mit 1 N Chlorwasserstoffsäure und dann mit gesättigter Natriumbicarbonatlösung gewaschen. Die organische Schicht wird über Magnesiumsulfat getrocknet und das

Lösungsmittel im Vakuum entfernt. Man erhält N-Propionyl-5,6-dihydro-4H-thieno[2,3-b]-thiopyran-5-amin. Dieses Produkt wird während 3 Stunden mit 85 ml 1 M Boran in Tetrahydrofuran unter Rückfluss erhitzt. Wasser und 3 N Chlorwasserstoffsäure wird zugefügt und das Gemisch noch kurz erhitzt. Die wässrige Schicht wird basisch gemacht, mit Aether extrahiert und das Lösungsmittel entfernt. Nach Zugabe von äthanolischer Chlorwasserstoffsäure erhält man N-Propyl-5,6-dihydro-4H-thieno-[2,3-b]thiopyran-5-amin-hydrochlorid, F.p. 107 - 110°.

Beispiel 5:

a) Ein Gemisch von 2,5 g N-Propyl-5,6-dihydro-4H-thieno[2,3-b]thiopyran-5-amin-hydrochlorid, 7,5 g Natriumcarbonat, 11 ml n-Butyljodid, 25 ml Toluol und 25 ml Wasser wird 3 Tage unter Rückfluss erhitzt. Nach Entfernung vom Lösungsmittel wird die organische Schicht mit äthanolischer Chlorwasserstoffsäure behandelt, man erhält N-Butyl-N-propyl-5,6-dihydro-4H-thieno[2,3-b]thiopyran-5-amin-hydrochlorid, F.p. 161 - 164°.

In analoger Weise werden hergestellt:

b) N-Isopropyl-N-propyl-5,6-dihydro-4H-thieno[2,3-b]thiopyran-5-amin-hydrobromid;

c) N-Isobutyl-N-propyl-5,6-dihydro-4H-thieno[2,3-b]thiopyran-5-amin-hydrobromid;

d) N-(2-Phenylethyl)-N-propyl-5,6-dihydro-4H-thieno[2,3-b]thiopyran-5-amin-hydrobromid.

Beispiel 6:

Ein Gemisch von 2,5 g N,N-Dipropyl-5,6-dihydro-4H-thieno[2,3-b]thiopyran-5-amin in 40 ml Tetrahydrofuran wird mit 4 ml 2,5 M n-Butyllithium in Hexan bei 0° behandelt. Nach 30 Minuten bei 0° wird 1,5 g Methyljodid zugegeben und dann das Gemisch eine Stunde bei Raumtemperatur gehalten. Nach Auflösung in Aether, Waschen mit Wasser wird der Rückstand getrocknet und das Lösungsmittel im Vakuum entfernt, man erhält 2-Methyl-N,N-dipropyl-5,6-dihydro-4H-thieno[2,3-b]thiopyran-5-amin.

Beispiel 7

Herstellung von 1.000 Kapseln mit 10,0 mg Wirkstoff:

| | |
|---|---|
| Bestandteile: N,N-Dipropyl-5,6-dihyro-4H-thieno[2,3-b]thiopyran-5-amin-hydrochlorid | 10,0 g |
| Lactose | 207,0 g |
| modifizierte Stärke | 80,0 g |
| Magnesiumstearat | 3,0 g |

Herstellung:

Das pulverige Material wird durch ein Sieb mit Oeffnungen von 6,0 mm Durchmesser gedrückt. Anschliesend mischt man in einem Mischer den Wirkstoff mit Magnesiumstearat, dann mit Lactose und der Stärke bis man eine homogene Masse erhält. Man füllt Nr. 2 Hardgelatinekapseln mit 300 mg der vorbereiteten Mischung.

## Ansprüche

1. Verbindungen der Formel I

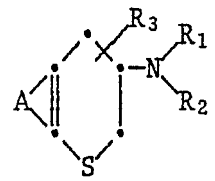

(I),

worin A einen divalenten Radikal $-S-CR_4=CR_5-$, worin $R_4$ und $R_5$ unabhängig voneinander Wasserstoff oder Niederalkyl, $R_1$ Wasserstoff, Niederalkyl oder Arylniederalkyl, $R_2$ Wasserstoff, Niederalkyl oder Arylniederalkyl, oder $R_1$ und $R_2$ zusammen Alkylen mit 4 bis 6 C-Atomen, und $R_3$ Wasserstoff oder Niederalkyl bedeuten; S-Oxide davon; und pharmazeutisch annehmbare Salze davon.

2. Verbindungen gemäss Anspruch 1 der Formel Ia

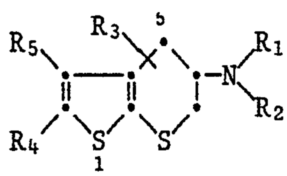

(Ia),

worin $R_1$ Wasserstoff, Niederalkyl oder Arylniederalkyl, $R_2$ Wasserstoff, Niederalkyl oder Arylniederalkyl, oder $R_1$ und $R_2$ zusammen Alkylen mit 4 bis 6 C-Atomen, $R_3$ Wasserstoff oder Niederalkyl, und $R_4$ und $R_5$ unabhängig voneinander Wasserstoff oder Niederalkyl bedeuten; S-Oxide davon; und pharmazeutisch annehmbare Salze davon.

3. Verbindungen gemäss Anspruch 1 der Formel Ib

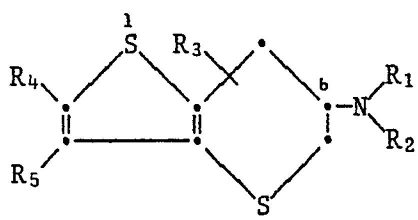

(Ib),

worin $R_1$ Wasserstoff, Niederalkyl oder Arylniederalkyl, $R_2$ Wasserstoff, Niederalkyl oder Arylniederalkyl, oder $R_1$ und $R_2$ zusammen Alkylen mit 4 bis 6 C-Atomen, $R_3$ Wassserstoff oder Niederalkyl, und $R_4$ und $R_5$ unabhängig voneinander Wasserstoff oder Niederalkyl bedeuten, S-Oxide davon, und pharmazeutisch annehmbare Salze davon.

4. Verbindungen gemäss Anspruch 1 der Formel I, worin $R_1$ und $R_2$ unabhängig voneinander Wasserstoff, Niederalkyl oder Arylniederalkyl, oder $R_1$ und $R_2$ zusammen Butylen oder Pentylen, und $R_3$, $R_4$ und $R_5$ Wasserstoff oder Niederalkyl bedeuten, und pharmazeutisch annehmbare Salze davon.

5. Verbindungen gemäss Anspruch 1 der Formel I, worin $R_1$ Niederalkyl, $R_2$ Niederalkyl oder Arylniederalkyl, und $R_3$, $R_4$ und $R_5$ Wasserstoff bedeuten, und pharmazeutisch annehmbare Salze davon.

6. Verbindungen gemäss Anspruch 1 der Formel I, worin $R_1$ und $R_2$ Niederalkyl, und $R_3$, $R_4$ und $R_5$ Wasserstoff bedeuten, und pharmazeutisch annehmbare Salze davon.

7. Verbindungen gemäss Anspruch 1 der Formel I, worin $R_1$ und $R_2$ geradkettiges $C_1$-$C_4$-Alkyl, und $R_3$, $R_4$ und $R_5$ Wasserstoff bedeuten, und pharmazeutisch annehmbare Salze davon.

8. Verbindungen gemäss Anspruch 2 der Formel Ia, worin $R_1$ und $R_2$ unabhängig voneinander Wasserstoff, Niederalkyl oder Arylniederalkyl, oder $R_1$ und $R_2$ zusammen Butylen oder Pentylen, und $R_3$, $R_4$ und $R_5$ Wasserstoff oder Niederalkyl bedeuten, und pharmazeutisch annehmbare Salze davon.

9. Verbindungen gemäss Anspruch 2 der Formel Ia, worin $R_1$ Niederalkyl, $R_2$ Niederalkyl oder Arylniederalkyl, und $R_3$, $R_4$ und $R_5$ Wasserstoff bedeuten, und pharmazeutisch annehmbare Salze davon.

10. Verbindungen gemäss Anspruch 2 der Formel Ia, worin $R_1$ und $R_2$ Niederalkyl, und $R_3$, $R_4$ und $R_5$ Wasserstoff bedeuten, und pharmazeutisch annehmbare Salze davon.

11. Verbindungen gemäss Anspruch 2 der Formel Ia, worin $R_1$ und $R_2$ geradkettiges $C_1$-$C_4$-Alkyl, und $R_3$, $R_4$ und $R_5$ Wasserstoff bedeuten, und pharmazeutisch annehmbare Salze davon.

12. N,N-Dipropyl-,5,6-dihydro-4H-thieno[2,3-b]thiopyran-5-amin und pharmazeutisch annehmbare Salze davon.

13. N,N-Dibutyl-5,6-dihydro-4H-thieno[2,3-b]thiopyran-5-amin und pharmazeutisch annehmbare Salze davon.

14. Pharmazeutische Präparate enthaltend Verbindungen der Ansprüche 1 - 13 mit einem pharmazeutisch geeigneten Trägermaterial.

15. Verwendung der Verbindungen gemäss den Ansprüchen 1 - 13 als präsynaptische Dopamin-Rezeptor Modulatoren.

16. Verwendung der Verbindung der Ansprüche 1 - 13 zur therapeutischen Behandlung von Dyskinesie, Parkinsonismus oder psychischen Erkrankungen.

17. Verfahren zur Herstellung von Verbindungen der Formel I gemäss Anspruch 1, worin alle Symbole die im Anspruch 1 angegebenen Bedeutungen haben, und deren pharmazeutisch annehmbaren Salzen, dadurch gekennzeichnet, dass man folgende Verfahrensvarianten ausführt:

a) Kondensation einer Verbindung der Formel II

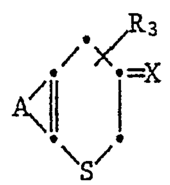

(II),

worin A und $R_3$ die im Anspruch 1 genannten Bedeutungen haben, X Sauerstoff oder reaktives verestertes Hydroxy zusammen mit Wasserstoff bedeutet, oder ein Mono-oder Di-S-Oxid einer Verbindung der Formel II, mit einer Verbindung der Formel III

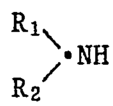

(III),

worin $R_1$ und $R_2$ die im Anspruch 1 genannten Bedeutungen haben; oder

b) Alkylierung einer Verbindung der Formel IV

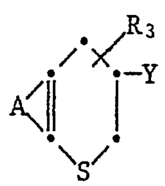

(IV),

worin A und $R_3$ die im Anspruch 1 genannten Bedeutungen haben, und Y -$NH_2$, $NHR_1$ oder $NHR_2$ bedeutet, oder ein Mono-oder Di-S-Oxid einer Verbindung der Formel IV mit einem reaktionsfähigen Ester eines Alkohols der Formel V

$R_1$-OH oder $R_2$-OH (V),

worin $R_1$ und $R_2$ die im Anspruch genannten Bedeutungen haben, oder mit einem $R_1$-OH und $R_2$-OH entsprechenden Aldehyd unter Einhaltung von reduzierenden Bedingungen; oder

c) Reduktion einer Verbindung der Formel VI

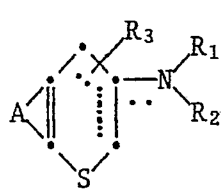

(VI),

worin $R_1$, $R_2$, $R_3$ und A die im Anspruch 1 genannten Bedeutungen haben, und die punktierten Bindungslinien eine Doppelbindung in einer der angezeigten Positionen darstellen, oder ein Mono-oder Di-S-Oxid einer Verbindung der Formel VI; oder

d) Reduktion einer Verbindung der Formel VII

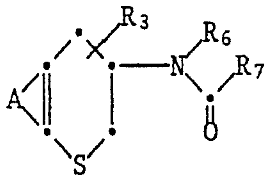

(VII),

worin $R_6$ $R_1$ oder $R_2$ bedeutet, A und $R_3$ die im Anspruch 1 genannten Bedeutungen haben; und $R_7$ Niederalkyl mit 1 - 6 C-Atomen, Arylniederalkyl mit 1 - 6 C-Atomen oder Wasserstoff bedeutet; oder ein Mono-oder Di-S-Oxid einer Verbindung der Formel VII; oder

e) Umwandlung eines Derivates, wie z.B. Azid-, primäres Amin-, N-Hydroxyamin-oder N-Acyloxyamin-Derivat, einer Carbonylsäure der Formel VIII

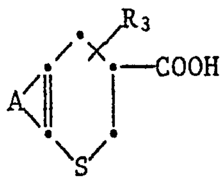

(VIII),

worin A und $R_3$ die im Anspruch 1 genannten Bedeutungen haben, oder eines mono-S-oder Di-S-Oxids einer Verbindung der Formel VIII, und eine Verbindung der Formel I, worin $R_1$ und $R_2$ Wasserstoff bedeuten isoliert; und gewünschtenfalls reaktive funktionelle Gruppen intermediär bei der Ausführung eines der genannten Verfahren schützt und anschliessend die freie Verbindung der Formel I isoliert und/oder eine erhaltene Verbindung der Formel I in eine andere Verbindung der Formel I umwandelt, und/oder eine erhaltene freie Verbindung in ein Salz oder ein Salz in die freie Verbindung oder in ein anderes Salz umwandelt, und/oder ein erhaltenes Gemisch von Isomeren oder Racematen in einzelne Isomere oder Racemate auftrennt, und/oder ein Racemat in die optischen Antipoden aufspaltet.

18. Die nach dem Verfahren des Anspruchs 17 erhältlichen Verbindungen.

Patentansprüche für die folgenden Vertragsstaaten:GR;ES

1. Verfahren zur Herstellung von Verbindungen der Formel I

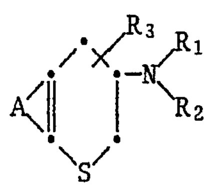

(I),

worin A einen divalenten Radikal -S-$CR_4$=$CR_5$-, worin $R_4$ und $R_5$ unabhängig voneinander Wasserstoff oder Niederalkyl, $R_1$ Wasserstoff, Niederalkyl oder Arylniederalkyl, $R_2$ Wasserstoff, Niederalkyl oder Arylniederalkyl, oder $R_1$ und $R_2$ zusammen Alkylen mit 4 bis 6 C-Atomen, und $R_3$ Wasserstoff oder Niederalkyl bedeuten; S-Oxide davon; und pharmazeutisch annehmbaren Salzen davon, dadurch gekennzeichnet, dass man folgende Verfahrensvarianten ausführt:

a) Kondensation einer Verbindung der Formel II

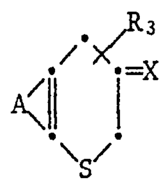

(II),

worin A und $R_3$ die oben genannten Bedeutungen haben, X Sauerstoff oder reaktives verestertes Hydroxy zusammen mit Wasserstoff bedeutet, oder ein Mono-oder Di-S-Oxid einer Verbindung der Formel II, mit einer Verbindung der Formel III

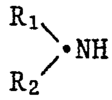

(III),

worin $R_1$ und $R_2$ die oben genannten Bedeutungen haben; oder

b) Alkylierung einer Verbindung der Formel IV

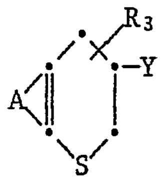

(IV),

worin A und $R_3$ die oben genannten Bedeutungen haben, und Y $-NH_2$, $NHR_1$ oder $NHR_2$ bedeutet, oder ein Mono-oder Di-S-Oxid einer Verbindung der Formel IV mit einem reaktionsfähigen Ester eines Alkohols der Formel V

$R_1$-OH oder $R_2$-OH (V),

worin $R_1$ und $R_2$ die oben genannten Bedeutungen haben, oder mit einem $R_1$-OH und $R_2$-OH entsprechenden Aldehyd unter Einhaltung von reduzierenden Bedingungen; oder

c) Reduktion einer Verbindung der Formel VI

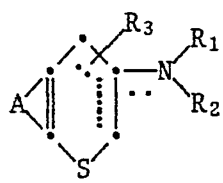

(VI),

worin $R_1$, $R_2$, $R_3$ und A die oben genannten Bedeutungen haben, und die punktierten Bindungslinien eine Doppelbindung in einer der angezeigten Positionen darstellen, oder ein Mono-oder Di-S-Oxid einer Verbindung der Formel VI; oder

d) Reduktion einer Verbindung der Formel VII

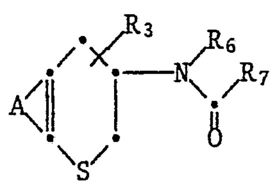

(VII),

worin $R_6$ $R_1$ oder $R_2$ bedeutet, A und $R_3$ die oben genannten Bedeutungen haben; und $R_7$ Niederalkyl mit 1 - 6 C-Atomen, Arylniederalkyl mit 1 - 6 C-Atomen oder Wasserstoff bedeutet; oder ein Mono-oder Di-S-Oxid einer Verbindung der Formel VII; oder

e) Umwandlung eines Derivates, wie z.B. Azid-, primäres Amin-, N-Hydroxyamin-oder N-Acyloxyamin-Derivat, einer Carbonylsäure der Formel VIII

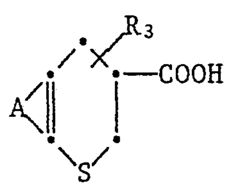

(VIII),

worin A und $R_3$ die oben genannten Bedeutungen haben, oder eines mono-S-oder Di-S-Oxids einer Verbindung der Formel VIII, und eine Verbindung der Formel I, worin $R_1$ und $R_2$ Wasserstoff bedeuten isoliert; und gewünschtenfalls reaktive funktionelle Gruppen intermediär bei der Ausführung eines der genannten Verfahren schützt und anschliessend die freie Verbindung der Formel I isoliert und/oder eine erhaltene Verbindung der Formel I in eine andere Verbindung der Formel I umwandelt, und/oder eine erhaltene freie Verbindung in ein Salz oder ein Salz in die freie Verbindung oder in ein anderes Salz umwandelt, und/oder ein erhaltenes Gemisch von Isomeren oder Racematen in einzelne Isomere oder Racemate auftrennt, und/oder ein Racemat in die optischen Antipoden aufspaltet.

2. Verfahren gemäss Anspruch 1, dadurch gekennzeichnet, dass man Verbindungen der Formel Ia

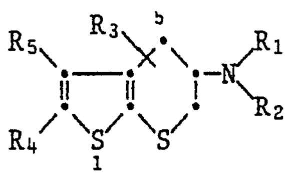

(Ia),

worin $R_1$ Wasserstoff, Niederalkyl oder Arylniederalkyl, $R_2$ Wasserstoff, Niederalkyl oder Arylniederalkyl, oder $R_1$ und $R_2$ zusammen Alkylen mit 4 bis 6 C-Atomen, $R_3$ Wasserstoff oder Niederalkyl, und $R_4$ und $R_5$ unabhängig voneinander Wasserstoff oder Niederalkyl bedeuten; S-Oxide davon; und pharmazeutisch annehmbare Salze davon herstellt.

3. Verfahren gemäss Anspruch 1, dadurch gekennzeichnet, dass man Verbindungen der Formel Ib

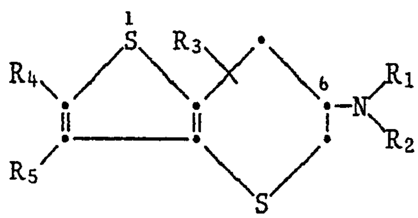

(Ib),

worin $R_1$ Wasserstoff, Niederalkyl oder Arylniederalkyl, $R_2$ Wasserstoff, Niederalkyl oder Arylniederalkyl, oder $R_1$ und $R_2$ zusammen Alkylen mit 4 bis 6 C-Atomen, $R_3$ Wasserstoff oder Niederalkyl, und $R_4$ und $R_5$ unabhängig voneinander Wasserstoff oder Niederalkyl bedeuten. S-Oxide davon, und pharmazeutisch annehmbare Salze davon, herstellt.

4. Verfahren gemäss Anspruch 1, dadurch gekennzeichnet, dass man Verbindungen der Formel I, worin $R_1$ und $R_2$ unabhängig voneinander Wasserstoff, Niederalkyl oder Arylniederalkyl, oder $R_1$ und $R_2$ zusammen Butylen oder Pentylen, und $R_3$, $R_4$ und $R_5$ Wasserstoff oder Niederalkyl bedeuten, und pharmazeutisch annehmbare Salze davon, herstellt.

5. Verfahren gemäss Anspruch 1, dadurch gekennzeichnet, dass man Verbindungen der Formel I, worin $R_1$ Niederalkyl, $R_2$ Niederalkyl oder Arylniederalkyl, und $R_3$, $R_4$ und $R_5$ Wasserstoff bedeuten, und pharmazeutisch annehmbare Salze davon, herstellt.

6. Verfahren gemäss Anspruch 1, dadurch gekennzeichnet, dass man Verbindungen der Formel I, worin $R_1$ und $R_2$ Niederalkyl, und $R_3$, $R_4$ und $R_5$ Wasserstoff bedeuten, und pharmazeutisch annehmbare Salze davon, herstellt.

7. Verfahren gemäss Anspruch 1, dadurch gekennzeichnet, dass man Verbindungen der Formel I, worin $R_1$ und $R_2$ geradkettiges $C_1$-$C_4$-Alkyl, und $R_3$, $R_4$ und $R_5$ Wasserstoff bedeuten, und pharmazeutisch annehmbare Salze davon, herstellt.

8. Verfahren gemäss Anspruch 1, dadurch gekennzeichnet, dass man Verbindungen der Formel Ia, worin $R_1$ und $R_2$ unabhängig voneinander Wasserstoff, Niederalkyl oder Arylniederalkyl, oder $R_1$ und $R_2$ zusammen Butylen oder Pentylen, und $R_3$, $R_4$ und $R_5$ Wasserstoff oder Niederalkyl bedeuten, und pharmazeutisch annehmbare Salze davon, herstellt.

9. Verfahren gemäss Anspruch 1, dadurch gekennzeichnet, dass man Verbindungen der Formel Ia, worin $R_1$ Niederalkyl, $R_2$ Niederalkyl oder Arylniederalkyl, und $R_3$, $R_4$ und $R_5$ Wasserstoff bedeuten, und pharmazeutisch annehmbare Salze davon, herstellt.

10. Verfahren gemäss Anspruch 1, dadurch gekennzeichnet, dass man Verbindungen der Formel Ia gemäss Anspruch 2, worin $R_1$ und $R_2$ Niederalkyl, und $R_3$, $R_4$ und $R_5$ Wasserstoff bedeuten, und pharmazeutisch annehmbare Salze davon, herstellt.

11. Verfahren gemäss Anspruch 1, dadurch gekennzeichnet, dass man Verbindungen der Formel Ia gemäss Anspruch 2, worin $R_1$ und $R_2$ geradkettiges $C_1$-$C_4$-Alkyl, und $R_3$, $R_4$ und $R_5$ Wasserstoff bedeuten, und pharmazeutisch annehmbare Salze davon, herstellt.

12. Verfahren gemäss Anspruch 1, dadurch gekennzeichnet, dass man N,N-Dipropyl-,5,6-dihydro-4H-thieno [2,3-b]thiopyran-5-amin oder pharmazeutisch annehmbare Salze davon herstellt.

13. Verfahren gemäss Anspruch 1, dadurch gekennzeichnet, dass man N,N-Dibutyl-5,6-dihydro-4H-thieno[2,3-b]thiopyran-5-amin oder pharmazeutisch annehmbare Salze davon herstellt.

14. Verfahren zur Herstellung eines pharmazeutischen Präparates, gekennzeichnet durch die Verarbeitung eines erfindungsgemässen Wirkstoffs nach einem der Ansprüche 1 - 13 mit einem pharmazeutisch annehmbaren Trägermaterial.

Europäisches Patentamt

# EUROPÄISCHER RECHERCHENBERICHT

Nummer der Anmeldung

EP 88 10 2715

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-1 908 496 (CIBA AG)<br>* Seite 7, Formel (Ia); Anspruch 6 * | 1-3 | C 07 D 495/04<br>A 61 K 31/38 //<br>(C 07 D 495/04<br>C 07 D 335:00<br>C 07 D 333:00 ) |
| A | US-A-4 092 325 (J.C. SIRCAR et al.)<br>* Anspruch 1 * | 1,2 | |
| A | US-A-4 082 757 (J.C. SIRCAR et al.)<br>* Anspruch 1 * | 1,2 | |
| A | CHEMICAL ABSTRACTS, Band 92, Nr. 5, 4. Februar 1980, Seite 771, Zusammenfassung Nr. 41801e, Columbus, Ohio, US; A.V. ANISIMOV et al.: "Cyclization of allylthiophenethiols", & KHIM. GETEROTSIKL. SOEDIN 1979, (8), 1062-1065 | 1,2 | |
| A | CHEMICAL ABSTRACTS, Band 94, nr. 17, 27. April 1981, Seite 737, Zusammenfassung Nr. 139536p, Columbus, Ohio, US; P. CAGNIANT et al.: "Synthesis, antifungal and antibacterial activity of aminoalkyl ketones in surfur heterocyclic series", & EUR. J. MED. CHEM. - CHIM. THER. 1980, 15(5), 439-447 | 1,3 | **RECHERCHIERTE SACHGEBIETE (Int. Cl.4)**<br>C 07 D 495/00 |

Der vorliegende Recherchenbericht wurde für alle Patentansprüche erstellt

| Recherchenort | Abschlußdatum der Recherche | Prüfer |
|---|---|---|
| BERLIN | 26-05-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument